# EUROPEAN PATENT SPECIFICATION

(11) **EP 1 959 933 B1**
(45) Date of publication and mention of the grant of the patent: **20.10.2010**
(21) Application number: 06830298.3
(22) Date of filing: 01.12.2006
(51) Int. Cl.: A61K 31/431, A61K 9/19

(54) **PROCESS FOR THE PREPARATION OF LYOPHILIZED PIPERACILLIN SODIUM IN COMBINATION WITH TAZOBACTAM SODIUM, WITH IMPROVED STABILITY AFTER RECONSTITUTION**
VERFAHREN ZUR HERSTELLUNG VON LYOPHILISIERTEM PIPERACILLIN-NATRIUM IN KOMBINATION MIT TAZOBACTAM-NATRIUM, MIT VERBESSERTER STABILITÄT NACH DER REKONSTITUTION
PROCÉDÉ POUR LA PRÉPARATION DE LA PIPÉRACILLINE SODIQUE LYOPHILISÉE EN COMBINAISON AVEC LE TAZOBACTAM SODIQUE, AYANT UNE MEILLEURE STABILITÉ APRÈS RECONSTITUTION

(30) Priority: 05.12.2005 US 742408 P
(43) Date of publication of application: 27.08.2008
(73) Proprietor: SANDOZ AG, 4056 Basel (CH)
(72) Inventor: DIAGO, Jose, E-08402 Granollers/Barcelona (ES); CABRE, Joan, E-08010 Barcelona (ES); SALVADOR, Josep, E-08015 Barcelona (ES); LLOVERAS, Pere, E-08221 Terrassa (ES); KOSILEK, Irina, A-6330 Kufstein (AT); ATZL, Norbert, A-6336 Langkampfen (AT)
(74) Representative: Wichmann, Hendrik
(86) International application number: PCT/EP2006/069232
(87) International publication number: WO 2007/065862

(56) References cited:
- GB-A- 2 231 266
- US-A1- 2004 204 372
- US-A1- 2005 171 077

## Description

### FIELD OF THE INVENTION

This invention relates to a process for the production of lyophilized Piperacillin Sodium in combination with Tazobactam Sodium.

Piperacillin in combination with Tazobactam is useful for intravenous administration as antibiotics for hospitalized patients with serious infections.

Specifically this invention relates to a process further including a control of the carbon dioxide content prior to lyophilization. The product obtained in this way has enhanced pH stability and therefore, enhanced resistance to particulate formation in solutions to be administered parenterally, with no need for additional components, such as buffering agents or chelating agents.

### BACKGROUND OF THE INVENTION

Piperacillin Sodium alone or in combination with Tazobactam Sodium is an antibiotic which is used in the treatment of moderate to severe infections caused by strains of microorganisms in conditions such as nosocomial pneumonia due to Staphylococcus aureus; intra-abdominal infections, specifically appendicitis and peritonitis due to Escherichia coli, skin and skin structure infections, including cellulitits, cutaneous abscesses and ischemic/diabetic foot infections due to Staphylococcus aureus; and gynecologic infections, specifically postpartum endometritis or pelvic inflammatory disease due to Escherichia coli. The seriousness of these infections highlights the need for a readily available and dependable treatment.

Polymicrobial infections often include pathogens that produce beta-lactamase enzymes. These enzymes commonly cause resistance to penicillins and cephalosporins. Without treatment these microbes would multiply and thrive unimpeded, with serious or critical consequences to the patient. Basically, Tazobactam permanently inactivates beta-lactamases, allowing Piperacillin to destroy susceptible bacteria.

Medicaments are formulated into not only emulsions, suspensions or solutions, but also as lyophilized preparations to be reconstituted before use.

Advantageously, lyophilized preparations are stable, can be stored and are easily reconstituted. Moreover lyophilized preparations may be kept sterile and essentially free of insoluble matter.

Piperacillin Sodium alone or in combination with Tazobactam Sodium is available as powder (lyophilized product) which is reconstituted by addition of a compatible reconstitution diluent prior to intravenous administration.

However when lyophilized Piperacillin Sodium alone or in combination with Tazobactam Sodium is reconstituted, particulate matter formation begins.

Particulates are formed as reconstituted lyophilized formulations are prepared and stored prior to patient administration. As the time increases from reconstitution and delivery to the patient so does formation of particulates. It is recognized that the presence of particles in solution, particularly if injected intravenously, can be harmful. In particular, it has been shown that the development of infusion-phlebitis may be related to the presence of particulate matter in intravenous fluids (Remmington's Pharmaceutical Sciences, 18 edition, Mack Publishing, 1990, page 1567).

When lyophilized Piperacillin Sodium alone or in combination with Tazobactam Sodium is reconstituted, the pH value of the solution decreases with time.

The pH decrease is caused mainly by degradation of Piperacillin in solution. Piperacillin is a beta-lactam product, known to be unstable in aqueous solutions.

As pH value decreases, the acid-base equilibrium for Piperacillin (a carboxylic acid) displaces to the acid form, resulting in a risk of crystallization of acid Piperacillin, which is very insoluble in water.

This pH decrease, and consequently the risk of particulate formation, may be reduced if either the solution is buffered prior to lyophilization or degradation is minimized.

For instance, U.S. Patent No. 6,207,661 discloses a citrate buffered Piperacillin, alone or in combination with Tazobactam, claiming for a superior stability.

U.S. Patent No. 6,900,184 discloses an EDTA containing Piperacillin alone or in combination with Tazobactam, EDTA acting as a chelating agent for zinc, which promotes degradation of Piperacillin; zinc ions coming from the plastic materials used in parenteral administration.

Parenteral drug products should be inspected visually for particulate matter prior to administration, whenever possible. It is desirable therefore to minimize the particulate formations that occur in the pharmaceutical compositions upon reconstitution.

Although the inventions cited might overcome the problem of particulate formation, the solutions disclosed involve the addition of other components with no therapeutic activity, i.e. buffer or chelating agents. It is desirable, therefore, to find a solution to this problem of particulate formation without any component other than the therapeutics products, i.e. Piperacillin and Tazobactam.

### BRIEF SUMMARY OF THE INVENTION

Surprisingly it has now been found that thoroughly removing carbon dioxide from the Piperacillin Sodium in combination with Tazobactam Sodium solution to low concentrations, prior to lyophilization, results in a consistent method of pH adjusting to the desired pH value, thus overcoming the problem of particulate formation after standing of the reconstituted solution.

Carbon dioxide remains in solution after the reaction involved in sodium salt formation (i.e. dissolving procedure), coming from sodium bicarbonate and / or carbonate plus acid Piperacillin and acid Tazobactam.

The removal of carbon dioxide is made by degassing; this degassing achieved by either vacuuming or gas blowing desorption, or a combination of both methods. After carbon dioxide removal, the pH can be adjusted, in a consistent way, to a value overcoming the risk of precipitation of the reconstituted solution.

Furthermore it has been unexpectedly found that a procedure for dissolving acid Piperacillin and acid Tazobactam by means of sodium bicarbonate and / or carbonate, different from that known by the prior art, results in minimized degradation, thus providing a product of better purity and, in addition, helping as well to minimize the problem of particulate formation after standing of the reconstituted solution.

### DETAILED DISCLOSURE

The adjustment of the pH value of the Piperacillin and Tazobactam solution prior to lyophilization is of utmost importance in order to avoid precipitation or particulate formation after reconstitution of the lyophilized product.

Although pharmacopoeial requirements for Piperacillin Sodium (United States Pharmacopoeia 28th Ed*.)* or for the combination of Piperacillin Sodium and Tazobactam Sodium *(*Pharmaceutical Forum, Pharmaceutical Previews, Vol. 28(6), Nov.-Dec. 2002*)* both allow for a pH value in the range from 5.0 to 7.0, it has been discovered that a pH value in the low segment, i.e. 5.0 to 5.5, leads unavoidably to a risk of precipitation of the reconstituted product after few hours.

This is due to the acid-base equilibrium displacement towards the acid form of Piperacillin, which is very insoluble in water.

Hence, it seems advisable to have a pH value in the high segment, i.e. 5.6 to 7.0.

In order to obtain a solution of Piperacillin Sodium in combination with Tazobactam Sodium from its corresponding acids, sodium bicarbonate and / or carbonate must be used, since other bases such as sodium hydroxide lead unavoidably to intensive degradation caused by side reactions.

Gaseous carbon dioxide evolves from solutions of Piperacillin Sodium with Tazobactam Sodium. Such solutions are prepared by reactions of the corresponding acids with sodium bicarbonate and / or carbonate. Such evolution is influenced by several factors, e.g. rate of addition, stirring speed, reaction container geometry, temperature, pressure. Variations in these factors lead obviously to variations in the dissolved carbon dioxide concentration. Even in strictly controlled experiments, relatively high differences in carbon dioxide concentration occur.

Unknown amounts of dissolved carbon dioxide interfere in the pH value of the lyophilized product in two ways:
a) The reaction is stopped at different extents
b) The pH reading is interfered

Point a) is easily understandable when considering the reaction equilibrium:

A-H + NaHCO₃ ⇆ A⁻ Na⁺ + CO₂ + H₂O,

*wherein A stands for either acid Piperacillin or acid Tazobactam*

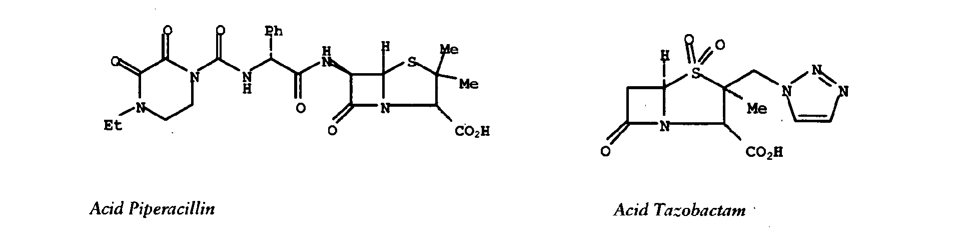

When carbon dioxide is evolved or removed to different unknown extents, the reaction progress to the right just to an accordingly unknown extent, and therefore the pH cannot be adjusted in a repetitive way, thus jeopardizing the final pH point to be adjusted to the desired value.

A related problem of erratic, unknown carbon dioxide content after dissolution is the considerable inertia observed, this inertia causing also erratic pH values of lyophilized products. This inertia is observed when adjusting pH, causing big variations with relatively small amounts of reactants added.

Point b) is related to the acidity conferred by dissolved carbon dioxide to the dissolution system. Therefore the pH measured has an unknown deviation from the true value, i.e. the value at carbon dioxide concentration near zero. As a result, the final pH value of the lyophilized product cannot be predicted as far as the carbon dioxide content is unknown, since carbon dioxide is almost completely removed after lyophilization.

With the present invention these problems are solved.

In one preferred embodiment of the present invention, acid Piperacillin and acid Tazobactam are reacted with sodium bicarbonate and / or carbonate in water as solvent; the solution of Piperacillin Sodium and Tazobactam Sodium thus obtained is degassed to a content of carbon dioxide of less than 200 mg/L, and pH adjusted to a value from 5.6 to 7.9.

In another preferred embodiment of the present invention, acid Piperacillin and acid Tazobactam are reacted with sodium bicarbonate in water as solvent; the solution of Piperacillin Sodium and Tazobactam Sodium thus obtained is degassed to a content of carbon dioxide of less than 75 mg/L, and pH adjusted to a value from 6.3 to 7.5.

Obviously the present invention may be applied to solutions of Piperacillin Sodium and Tazobactam Sodium prepared from one product in acid form and the other in already sodium salt form. For instance a solution may be prepared by dissolving Piperacillin Sodium in water and then reacting acid Tazobactam with sodium bicarbonate or *vice versa.*

Noticeably the present invention may be applied to buffered solutions of Piperacillin Sodium in combination with Tazobactam Sodium. For instance sodium citrate may be added during or after the reaction of Piperacillin in combination with Tazobactam with sodium bicarbonate and/or sodium carbonate. Other substances or additives, such as a particulate formation inhibitor, may be added as well.

Degassing may be accomplished by means of vacuum and/or bubbling of a gas through the solution.

Bubbling of nitrogen, for instance, may be applied throughout the addition step, thus helping carbon dioxide to escape from the solution.

Preferably vacuum is applied after the addition step, and more preferably it is maintained during all post-addition reaction time until filtration of the solution is started.

Vacuum may be applied to an absolute pressure of less than 300 mBar, preferably less than 100 mBar, and more preferably less than 30 mBar.

The measurement of carbon dioxide concentration may be made by any suitable analytical method, preferably by means of a selective electrode.

When reacting acid Piperacillin and acid Tazobactam with sodium bicarbonate and / or carbonate, several possible combinations exist, regarding the order of addition of these components. The addition might be simultaneous, but the formed foam would render the process not practical.

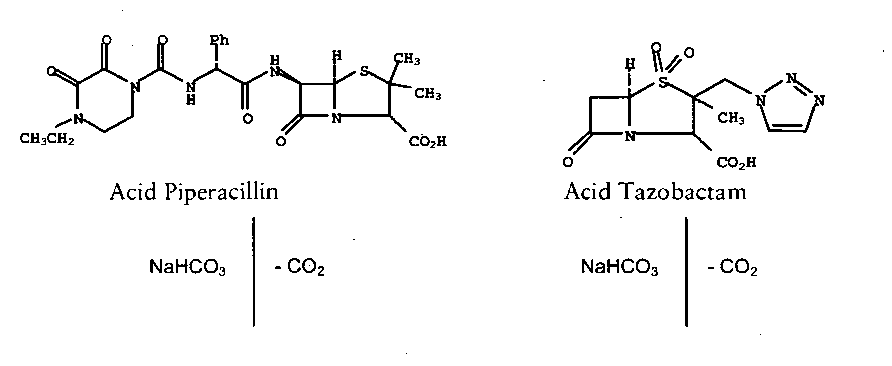

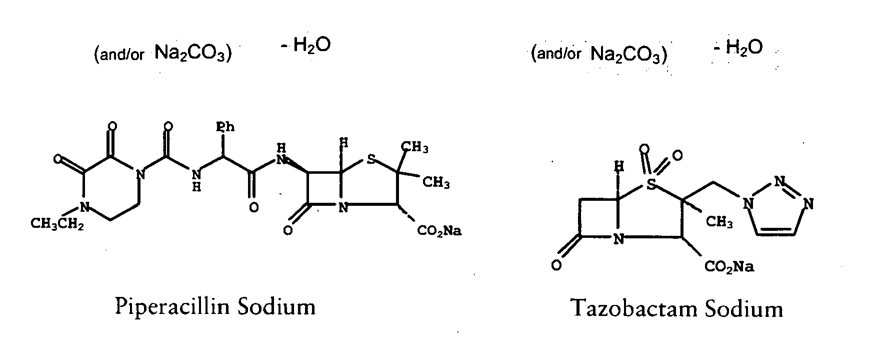

Three possible combinations have been investigated:
A) Sodium bicarbonate and / or carbonate over acid Piperacillin and acid Tazobactam suspended in water
B) Acid Piperacillin over sodium bicarbonate and / or carbonate dissolved in water and then acid Tazobactam over the whole
C) Sodium bicarbonate and / or carbonate, acid Piperacillin and acid Tazobactam over water, alternate

For each alternative, several temperature, stirring speed and time conditions have been investigated.

The procedure for reacting acid Piperacillin and acid Tazobactam with sodium bicarbonate known in the prior art (US Patent No. 6,900,184), is carried out by the addition of sodium bicarbonate over the acid Piperacillin and acid Tazobactam suspended in water. It corresponds, therefore, to method A).

This is the most apparent method of addition, in theory, since there is never a high pH due to free sodium bicarbonate - higher pH causing a higher degradation rate.

Nevertheless, some disadvantages are revealed after careful studies:
◆ Solution is not complete
◆ It cannot be driven at low temperatures
◆ Strong foam formation is unavoidable.

The first issue is important because it is the source for potential problems of inertia in the pH adjustment. When acid Piperacillin reacts with limited amounts of sodium bicarbonate and /or carbonate, as in the ultimate part of the reaction, the later is very slow.

It has now been found that if this reaction is carried out at temperatures of 15°C or below, the reaction is so slow that a lot of product remains undissolved even after long periods of stirring.

In addition, it has been found, as it would be expected, that degradation of already dissolved products, i.e. Piperacillin and Tazobactam is considerably faster at temperatures near room temperatures than at lower temperatures. After stability studies, an inflexion point around 15°C has been determined.

Hard foam formation is unavoidable with this method, since there is a lot of insoluble material, i.e. acid Piperacillin and Tazobactam, throughout the addition step; acid Piperacillin is a very hydrophobic product, thus contributing to foam formation and stabilization.

The volume of generated foam may be as high as four times the original volume of suspension. This is a clear disadvantage from the industrial point of view, since there is a need for a large reaction vessel and moreover a risk of overflowing of material out of the vessel.

After careful investigations it has been determined method B) as the best alternative. It has the following advantages:
◆ It may be driven at different temperatures, including low temperatures.
◆ Solution is complete '
◆ Foam formation is smooth

Thus the disadvantages intrinsic to the prior art are overcome:

The process may be carried out at low temperatures, thus minimizing degradation.

The solution is complete, thus overcoming the issue related to inertia in pH adjustment and, in addition easing the filtration step, needed to render the solution sterile prior to the lyophilization step.

A parenteral product, such as the object of the present invention, namely Piperacillin /Tazobactam, obviously has to be sterile, and this is accomplished by means of a sterile filtration whilst the product is in solution.

Nevertheless, the present invention is appropriate indeed for the manufacturing of oral, non-sterile, product.

Foam formation is in this case a much less problem, since there is no presence of large amounts of insoluble products at anytime. The maximum observed height of foam is twice the original volume, thus reducing the volume observed with the method known by the prior art to the half. What is more, the persistence of foam, i.e. the dead time between solid portion additions, is considerably reduced.

Method C) is similar to B), but with slight disadvantages, concerning easiness of operation.

Thus, a process for the production of lyophilized Piperacillin Sodium, in combination with Tazobactam Sodium, with improved stability of the reconstituted solution is provided, comprising the steps of:
i. Dissolving acid Piperacillin, alone or in combination with Tazobactam, in water by means of reaction with sodium bicarbonate and / or sodium carbonate.
ii. Degassing until a carbon dioxide content of less than 200 mg /L
iii. pH adjustment to a value between 5.6 and 7.9
iv. Lyophilization

In a preferred embodiment of the present invention, sodium bicarbonate and / or carbonate is dissolved in water, acid Piperacillin is added over the base as solid portions and then acid Tazobactam is added as solid portions.

The relative amounts of reactants are approximately equimolar, preferably using a range of molar relationship of sodium bicarbonate and / or carbonate with respect to the sum of both acid Piperacillin and acid Tazobactam of 5% excess to 5% defect of sodium bicarbonate, more preferably of 2% excess to 2% defect and even more preferably of 1% excess to 1% defect.

Sodium bicarbonate is preferred to sodium carbonate since the former solutions have lower pH values and, therefore, less degradation is caused.

Nevertheless, sodium carbonate may be used, in combination or alone with the aim to speed the dissolving process.

For instance, after the addition of Piperacillin is started and hence the pH is somewhat buffered, an effective amount of sodium carbonate may be added, provided the total molar ratio of base is in the range stated before.

The Piperacillin to Tazobactam ratio is in the range from 20:1 to 1:1, preferably 8:1 and 4:1, expressed as potency of anhydrous acids in the lyophilized product.

Noticeably the present invention may be applied separately to both products, that is to say, single Piperacillin Sodium and single Tazobactam Sodium.

The temperature of the dissolving stage is in the range of 40°C to -5°C, preferably in the range of 15°C to 5°C and more preferably of 9°C to 5°C.

For instance sodium bicarbonate and / or carbonate may be dissolved at 40°C and the solution cooled to less than 15°C, preferably less than 10°C, and prior to the addition of acid Piperacillin.

Addition of acid Piperacillin may be as quick as possible, regarding foam formation.

Advantageously, the first added portion of acid Piperacillin should be more than 5%, preferably more than 10%, of the total amount to be added, in order to get a fairly buffered system at once.

After addition of all acid Piperacillin, the whole may be stirred for a period of time, for instance 15 or 30 minutes, and then addition of acid Tazobactam is started immediately,

During addition of solids, i.e. acid Piperacillin and acid Tazobactam, any suitable gas, preferably nitrogen, may be bubbled for degassing purposes. Alternatively vacuum may be applied within the same step, continuously or intermittently with the same aim.

Having added all solids, the reaction is let for completion for a period of time, for at least 10 minutes, preferably for at least 20 minutes, and more preferably for a period of 30 to 60 minutes.

During this post-addition time, either nitrogen bubbling and / or vacuum are applied for the scope of degassing.

After this post-addition time the carbon dioxide content is checked to be less than 200 mg/L, preferably less than 100 mg/L and more preferably less than 75 mg/L.

If the carbon dioxide content is higher than wanted, degassing is continued for an extra time, 15 to 30 minutes for instance, the carbon dioxide content checked again and degassing repeated until the carbon dioxide content below the sought value.

Once the carbon dioxide content is below the desired value, the pH value is checked to be in the range 5.6 to 7.7, preferably 6.0 to 7.3 and more preferably 6.3 to 7.1.

If the pH value is lower than wanted, an additional small amount of sodium bicarbonate and / or carbonate is added; reversely, if the pH value is higher than wanted, an additional small amount of acid Piperacillin and / or acid Tazobactam is added instead; the whole is let to react for a period of time, 15 to 30 minutes for instance, during which time degassing may be carried out, the pH value checked again and the pH adjustment repeated until the pH value is in the sought range.

The pH adjusted in this way corresponds to a pH value after lyophilization in the range 5.6 to 7.0, thus avoiding or minimizing the risk of precipitation or particulate formation of the reconstituted solution.

The solution thus obtained may be sterile filtered, for instance through a 0.2 or 0.1 micron sterilizing cartridge, and then lyophilized by methods known in prior art.

Any suitable lyophilization recipe may be used. For instance the solution is frozen to less than -30°C, vacuum is applied to less than 1 mBar and then the frozen cake is heated up to 60°C until dryness.

The lyophilization may be carried out in vials or as bulk in trays.

The product obtained according to the present invention has improved stability of reconstituted solution. For instance, the product is dissolved in water and let stand for 24 hours at room temperature: neither precipitation nor opalescence is observed. Concentration of reconstituted solution is usually between 15 and 25%, more frequently around 20%.

The analysis of impurities coming from degradation substantiates the stability in solution.

The following Table summarizes the degradation of the product obtained according to the present invention with respect to that obtained according to prior art.

| | Total Impurities - Typical values | |
|---|---|---|
| | Initial | After 24h in solution |
| Present invention | 0.50% | 1.33% |
| Marketed Sample | 1.35% | 2.76% |

Further, the product prepared by the process of the present invention provides clear advantage over the commercially available product regarding the behavior on reconstitution. Both products show a similar dissolution time, but the commercially available product develops a strong foam formation during dissolution. Whereas the product of the present invention dissolves to form a clear solution which is ready for use, the commercially available product needs several additional minutes until the foam disappears, thus more than doubling the toal time to application.

Foam formation of the commercially available product on reconstitution does not allow a clear evaluation if all product is dissolved or if some particles are still in the foam. Therefore, for security reasons the time to application has to be the total dissolution time which is the dissolution time to a clear solution extended by the foam disappearing time. Moreover, the product obtained according to the present invention allows a quicker and more exact dosage than a product suffering from strong foam formation on reconstitution.

| | Dissolution time | Foam disappearing time | Total time to application |
|---|---|---|---|
| | [min] | [min] | [min] |
| Present invention | 01:05 - 02:25 | 00:00 | 01:05 - 02:25 |
| Marketed samples | 01:10 - 01:55 | 03:00 - 04:00 | 04:10 - 05:25 |

The product obtained according to the present invention containing a lyophilized mixture of Piperacillin sodium and Tazobactam sodium is adjusted to a water content of below 1.0% (w/w) by drying means and ensuring by sieving that the amount of small particles (< 50µm) is not more than 50%. A pharmaceutical composition containing this product has a low tendency to stick on the vial bottom during reconstitution, has a fast dissolution time and shows no foam formation.

### Subvisible particles

Subvisible particle content (particle sizes ≥ 10µm or ≥ 25µm) is a critical aspect in pharmaceutical compositions designated for intravenous applications. US P 6900184 asserts that conventional Piperacillin Sodium / Tazobactam Sodium preparations have unsatisfactory results with regard to particulate matter formation if not formulated with an aminocarboxylic acid chelating agent, especially when used together with aminoglycoside antibiotics. Surprisingly the product obtained according to the present invention as well as the marketed sample show no critical content of subvisible particles when reconstituted and transferred in an infusion bag with or without filtration. No impact of an aminocarboxlic acid chelating agent (EDTA) or presence of an aminoglycoside antibiotic (amikacin) is observed. Particles are determined at the beginning (0 h) and after 24 h.

| **preparation** | | **Present invention** | | **Marketed sample** | |
|---|---|---|---|---|---|
| | | **2,25 g** | | **2,25 g** | |
| | | **0 h** | **24 h** | **0 h** | **24 h** |
| | | **particles/5ml** | | **particles/5ml** | |
| Vial + 10 ml EDTA Solution + 10 ml water + Amikacin | > 10 µm | *328* | *38* | *776* | *110* |
| no filtration into the bag | > 25 µm | *3* | *4* | *15* | *16* |
| Vial + 10 ml EDTA Solution + 10 ml water + Amikacin | > 10 µm | *61* | *35* | *61* | *29* |
| with filtration into the bag | > 25 µm | *8* | *9* | *7* | *13* |
| 1 Vial + 20 ml water + Amikacin | > 10 µm | *491* | *104* | *636* | *179* |
| no filtration into the bag | > 25 µm | *3* | *6* | *10* | *10* |
| 1 Vial + 20 ml water + Amikacin | > 10 µm | *46* | *69* | *32* | *28* |
| with filtration into the bag | > 25 µm | *2* | *23* | *3* | *7* |
| 1 Vial + 20 ml 0,9% NaCl + Amikacin | > 10 µm | *21* | *71* | *31* | *42* |
| with filtration into the bag | > 25 µm | *4* | *18* | *3* | *9* |

A pharmaceutical composition containing the product obtained according to the present invention has no need to be formulated with additional auxiliaries. The reconstituted solutions using different diluents show excellent stability and satisfactory results in terms of subvisible particles even after transferring the reconstituted solution from a vial into an infusion bag and stored for a 7 day period.

Infusion bags (total of 160 mL solution) stored 7 days at 2 - 8 °C

| | | | | Subvisible Particles | |
|---|---|---|---|---|---|
| Batch | Diluent | | pH | ≥ 10 µm/5 mL | ≥ 25 µm/5 mL |
| Present invention (2.25g vial) | 0.9% Sodium Chloride for Injection | 0 days | 4.8 | 29 | 0 |
| | | 7 days | 4.6 | 7 | 1 |
| | Lactated Ringer's | 0 days | 5.6 | 27 | 0 |
| | Solution | 7 days | 5.4 | 11 | 1 |
| | Dextrose 5 % | 0 days | 5.0 | 33 | 1 |
| | | 7 days | 4.7 | 15 | 1 |
| | Dextran 6% in | 0 days | 4.9 | 73 | 2 |
| | Saline | 7 days | 4.6 | 13 | 0 |
| | | | | | |
| Marketed sample (2.25g vial) | 0.9% Sodium Chloride for Injection | 0 days | 4.9 | 162 | 5 |
| | | 7 days | 4.6 | 12 | 1 |
| | Lactated Ringer's | 0 days | 5.8 | 102 | 1 |
| | Solution | 7 days | 5.5 | 5 | 0 |
| | Dextrose 5% | 0 days | 5.1 | 226 | 7 |
| | | 7 days | 4.8 | 7 | 0 |
| | Dextran 6% in | 0 days | 5.0 | 244 | 5 |
| | Saline | 7 days | 4.7 | 50 | 3 |

All subvisible particle determinations are performed according to US Pharmakopoeia.

### EXPERIMENTAL METHODS

### Stability of Solution Test (SST)

2.35 g of Piperacillin Sodium or 2.35 g of Piperacillin Sodium / Tazobactam Sodium 8:1 combination is placed in a 30 mL vial. 10.0 mL of water are added, the vial stopped and shaken until total solution.

The vial is let stand for 24 hours at room temperature; after this period the vial is observed for any insoluble material.

Results Key:
T: transparent
O: opalescent
P: precipitated

### Dissolution Time, Foam Formation Test

Piperacillin Sodium / Tazobactam Sodium 8:1 lyophilised powder of different strengths (2.25g, 3.375g, 4,5g) is placed in vials. The powder is shaken up and equivalent amounts of water (10, 15, 20 ml) are added to the vials via syringe. The time until a clear solution is observed is measured.

Vials of marketed samples are shaken up and equivalent amounts of water (10, 15, 20 ml) are added to the vials via syringe. The time until a clear solution is observed is measured. The time until the foam formed during dissolution disappears is measured.

Dissolution time with regard to particle size: Piperazillin Sodium / Tazobactam Sodium 8:1 lyophilised powder is sieved into three fractions: <50µm, 50-100µm, >100µm. 3.375g of these fractions are placed in vials and dissolved with 15 ml water.

### Carbon Dioxide Measurement

A combined pH-meter / ionometer is used.
(MultiSeven, Mettler-Toledo; reference CO₂ electrode: 51341200)

The ionometer is calibrated in the range 1 - 1000 mg CO₂/L.

### Reference Example - No degassing

In a 1L stirred reactor, 109 mL of water, 60.00 g of Piperacillin (11.2 cMol) Monohydrate and 7.24 g (2.4 cMol)of Tazobactam are loaded. With good stirring 11.43 g (13.6 cMol) of sodium bicarbonate are loaded in portions over a period of 1 hour. Stirring is continued for 30 minutes and pH adjusted to a value between 5.5 and 7.0 by means of Piperacillin Monohydrate (PIP) or Sodium Bicarbonate (BIC) addition, as needed. The solution is then lyophilized.

| Initial pH | Adjust | Adjusted pH | Final pH | SST |
|---|---|---|---|---|
| 7.2 | 0.2 g PIP | 6.2 | 5.2 | P |

Initial pH: before pH adjusting
Final pH: after lyophilization,
(according to United States Pharmacopoeia, 28th Edition)

### Example 1

In a 1L stirred reactor, 109 mL of water, 60.00 g of Piperacillin Monohydrate (equivalent to 58.00 g of anhydrous acid; 11.2 cMol) and 7.25 g (2.4 cMol) of Tazobactam (Piperacillin to Tazobactam ratio 8:1) are loaded. With good stirring 11.43 g of sodium bicarbonate (13.6 cMol) are loaded in portions over a period of 1 hour. Stirring is continued for 30 minutes while vacuum is applied to an absolute pressure of 20-30 mBar. CO₂ is measured to be less than 75 mg/L (if higher, stirring under vacuum is continued for additional 30 minutes) and pH adjusted to a value between 5.5 and 7.0 by means of Piperacillin Monohydrate (PIP) or Sodium Bicarbonate (BIC) addition, as needed. The solution is then lyophilized.

| Initial pH | mg CO₂/L | Adjust | Adjusted pH | Final pH | SST |
|---|---|---|---|---|---|
| 7.8 | 72 | 0.4 g PIP | 6.8 | 6.5 | T |

### Example 2

In a 1L stirred reactor, 109 mL of water and 11.43 g of sodium bicarbonate (13.6 cMol) are loaded. Temperature is lowered to a value between 6 and 9°C. With good stirring 60.00 g of Piperacillin Monohydrate (equivalent to 58.00 g of anhydrous acid; 11.2 cMol) and 7.25 g (2.4 cMol) of Tazobactam (Piperacillin to Tazobactam ratio 8:1) are loaded in portions over a period of 1 hour. Stirring is continued for 30 minutes while vacuum is applied to an absolute pressure of 20-30 mBar. CO₂ is measured to be less than 75 mg/L (if higher, stirring under vacuum is continued for additional 30 minutes) and pH adjusted to a value between 5.5 and 7.0 by means of Piperacillin Monohydrate (PIP) or Sodium Bicarbonate (BIC) addition, as needed. The solution is then lyophilized.

| Initial pH | mg CO₂ /L | Adjust | Adjusted pH | Final pH | SST |
|---|---|---|---|---|---|
| 7.5 | 67 | 0.4 g PIP | 6.6 | 6.3 | T |

### Reference example 3

In a 1L stirred reactor, 109 mL of water and 11.43 g of sodium bicarbonate (13.6 cMol) are loaded. Temperature is lowered to a value between 6 and 9°C. With good stirring 72.90 g (13.6 cMol) of Piperacillin Monohydrate are loaded in portions over a period of 1 hour. Stirring is continued for 30 minutes while vacuum is applied to an absolute pressure of 20-30 mBar. CO₂ is measured to be less than 75 mg/L (if higher, stirring under vacuum is continued for additional 30 minutes) and pH adjusted to a value between 5.5 and 7.0 by means of Piperacillin Monohydrate (PIP) or Sodium Bicarbonate (BIC) addition, as needed. The solution is then lyophilized.

| Initial pH | mg CO₂/L | Adjust | Adjusted pH | Final pH | SST |
|---|---|---|---|---|---|
| 7.9 | 61 | 0.5 g PIP | 6.9 | 6.6 | T |

### Reference example 4

In a 1L stirred reactor, 109 mL of water, 10.33 g (13.0 cMol) of sodium bicarbonate and 0.69 g (0.3 cMol) of sodium carbonate are loaded. Temperature is lowered to a value between 6 and 9°C. With good stirring 72.90 g (13.6 cMol) of Piperacillin Monohydrate are loaded in portions over a period of 1 hour. Stirring is continued for 30 minutes while vacuum is applied to an absolute pressure of 20-30 mBar. CO₂ is measured to be less than 75 mg/L (if higher, stirring under vacuum is continued for additional 30 minutes) and pH adjusted to a value between 5.5 and 7.0 by means of Piperacillin Monohydrate (PIP) or Sodium Bicarbonate (BIC) addition, as needed. The solution is then lyophilized.

| Initial pH | mg CO₂/L | Adjust | Adjusted pH (before lyophilization) | Final pH (after lyophilization) | SST |
|---|---|---|---|---|---|
| 8.1 | 64 | 0.6 g PIP | 6.8 | 6.4 | T |

### Example 5

In a 1L stirred reactor, 120 mL of water, 11.43 g of sodium bicarbonate (13.6 cMol) and 2.54 g (0.1 cMol) of sodium citrate are loaded. Temperature is lowered to a value between 6 and 9°C. With good stirring 60.00 g of Piperacillin Monohydrate (equivalent to 58.00 g of anhydrous acid; 11.2 cMol) and 7.25 g (2.4 cMol) of Tazobactam (Piperacillin to Tazobactam ratio 8:1) are loaded in portions over a period of 1 hour. Stirring is continued for 30 minutes while vacuum is applied to an absolute pressure of 20-30 mBar. CO₂ is measured to be less than 75 mg/L (if higher, stirring under vacuum is continued for additional 30 minutes) and pH adjusted to a value around 6.5 by means of Piperacillin Monohydrate (PIP) or Sodium Bicarbonate (BIC) addition, as needed. The solution is then lyophilized.

| Initial pH | mg CO₂/L | Adjust | Adjusted pH | Final pH | SST |
|---|---|---|---|---|---|
| 6.4 | 52 | - | 6.4 | 6.5 | T |

### Example 6

In a 1L stirred reactor, 121 mL of water and 12.60 g of sodium bicarbonate (15.0 cMol) are loaded. Temperature is lowered to a value between 6 and 9°C. With good stirring 60.00 g of Piperacillin Monohydrate (equivalent to 58.00 g of anhydrous acid; 11.2 cMol) and 14.50 g (4.8 cMol) of Tazobactam (Piperacillin to Tazobactam ratio 4:1) are loaded in portions over a period of 1 hour. Stirring is continued for 30 minutes while vacuum is applied to an absolute pressure of 20-30 mBar. CO₂ is measured to be less than 75 mg/L (if higher, stirring under vacuum is continued for additional 30 minutes) and pH adjusted to a value between 5.5 and 7.0 by means of Piperacillin Monohydrate (PIP) or Sodium Bicarbonate (BIC) addition, as needed. The solution is then lyophilized.

| Initial pH | mg CO₂/L | Adjust | Adjusted pH | Final pH | SST |
|---|---|---|---|---|---|
| 8.0 | 58 | 0.5 g PIP | 6.9 | 6.6 | T |

### Example 7

In a 1L stirred reactor, 121 mL of water, 10.08 g of sodium bicarbonate (12.0 cMol) and 1.59 g of sodium carbonate (1.5 cMol; 3.0 cEq) are loaded. Temperature is lowered to a value between 6 and 9°C. With good stirring 60.00 g of Piperacillin Monohydrate (equivalent to 58.00 g of anhydrous acid; 11.2 cMol) and 14.50 g (4.8 cMol) of Tazobactam (Piperacillin to Tazobactam ratio 4:1) are loaded in portions over a period of 1 hour. Stirring is continued for 30 minutes while vacuum is applied to an absolute pressure of 20-30 mBar. CO₂ is measured to be less than 75 mg/L (if higher, stirring under vacuum is continued for additional 30 minutes) and pH adjusted to a value between 5.5 and 7.0 by means of Piperacillin Monohydrate (PIP) or Sodium Bicarbonate (BIC) addition, as needed. The solution is then lyophilized.

| Initial pH | mg CO₂/L | Adjust | Adjusted pH | Final pH | SST |
|---|---|---|---|---|---|
| 7.9 | 66 | 0.4 g PIP | 6.8 | 6.5 | T |

## Claims

1. A process for the production of lyophilized Piperacillin Sodium in combination with Tazobactam Sodium, with improved stability of the reconstituted solution, comprising the steps of:
i. Dissolving acid Piperacillin in combination with Tazobactam in water by means of reaction with sodium bicarbonate and / or sodium carbonate.
ii. Degassing until a carbon dioxide content of less than 200 mg / L
iii. pH adjustment to a value between 5.6 and 7.9
iv. Lyophilization

2. The process according to claim 1 further comprising a sterile filtration prior to lyophilisation.

3. The process according to any of the claims 1 to 2 wherein approximately equivalent molar amounts of base, namely sodium bicarbonate and / or sodium carbonate, with respect to acids, namely acid Piperacillin in combination with acid Tazobactam, are used.

4. The process according to any of the claims 1 to 2 wherein approximately equivalent molar amounts of sodium bicarbonate with respect to acid Piperacillin and acid Tazobactam are used.

5. The process according to any of the claims 1 to 4 wherein the acids, namely acid Piperacillin in combination with Tazobactam, are added over the base, namely sodium bicarbonate and / or sodium carbonate, dissolved in water at a temperature not exceeding 15°C.

6. The process according to any of the claims 1 to 4 wherein acid Piperacillin and acid Tazobactam are added over sodium bicarbonate dissolved in water at a temperature between 5 and 9°C.

7. The process according to any of the claims 1 to 6 wherein degassing until a carbon dioxide content of less than 75 mg / L is applied.

8. The process according to any of the claims 1 to 6 wherein degassing is achieved by means of vacuum at an absolute pressure lower than 100 mBar.

9. The process according to any of the claims 1 to 6 wherein degassing is achieved by means of vacuum at an absolute pressure lower than 30 mBar.

10. The process according to any of the claims 1 to 6 wherein pH is adjusted to a value between 6.3 and 7.1.

11. The process according to any of the claims 1 to 10 wherein the ratio of Piperacillin to Tazobactam is in the range from 20:1 to 1:1.

12. The process according to any of the claims 1 to 11 wherein the ratio of Piperacillin to Tazobactam is 8:1.

13. The process according to any of the claims 1 to 11 wherein the ratio of Piperacillin to Tazobactam is 4:1.

## Patentansprüche

1. Verfahren für die Herstellung von lyophilisiertem Piperacillin-Natrium in Kombination mit Tazobactam-Natrium, mit verbesserter Stabilität der rekonstitutierten Lösung, umfassend die Schritte:
i. Lösen von Piperacillin-Säure in Kombination mit Tazobactam in Wasser mittels Umsetzung mit Natriumbicarbonat und/oder Natriumcarbonat
ii. Entgasen bis auf einen Kohlendioxidgehalt von weniger als 200 mg/L
iii. pH-Einstellung auf einen Wert zwischen 5,6 und 7,9
iv. Lyophilisierung.

2. Verfahren nach Anspruch 1, ferner umfassend eine Sterilfiltration vor der Lyophilisierung.

3. Verfahren nach einem beliebigen der Ansprüche 1 bis 2, wobei ungefähr äquivalente molare Mengen an Base, nämlich Natriumbicarbonat und/oder Natriumcarbonat in Bezug auf Säuren, nämlich Piperacillin-Säure in Kombination mit Tazobactam-Säure, verwendet werden.

4. Verfahren nach einem beliebigen der Ansprüche 1 bis 2, wobei ungefähr äquivalente molare Mengen an Natriumbicarbonat in Bezug auf Piperacillin-Säure und Tazobactam-Säure verwendet werden.

5. Verfahren nach einem beliebigen der Ansprüche 1 bis 4, wobei die Säuren, nämlich Piperacillin-Säure in Kombination mit Tazobactam, zu der Base, nämlich Natriumbicarbonat und/oder Natriumcarbonat, gelöst in Wasser, bei einer Temperatur, die nicht 15 °C übersteigt, hinzugefügt werden.

6. Verfahren nach einem beliebigen der Ansprüche 1 bis 4, wobei Piperacillin-Säure und Tazobactam-Säure zu Natriumbicarbonat, gelöst in Wasser, bei einer Temperatur zwischen 5 und 9 °C hinzugefügt werden.

7. Verfahren nach einem beliebigen der Ansprüche 1 bis 6, wobei das Entgasen bis zu einem Kohlendioxidgehalt von weniger als 75 mg/l durchgeführt wird.

8. Verfahren nach einem beliebigen der Ansprüche 1 bis 6, wobei das Entgasen mittels Vakuum bei einem absoluten Druck, der niedriger als 100 mBar ist, erreicht wird.

9. Verfahren nach einem beliebigen der Ansprüche 1 bis 6, wobei das Entgasen mittels Vakuum bei einem absoluten Druck von weniger als 30 mBar erreicht wird.

10. Verfahren nach einem beliebigen der Ansprüche 1 bis 6, wobei der pH auf einen Wert zwischen 6,3 und 7,1 eingestellt wird.

11. Verfahren nach einem beliebigen der Ansprüche 1 bis 10, wobei das Verhältnis von Piperacillin zu Tazobactam in dem Bereich von 20:1 bis 1:1 1 ist.

12. Verfahren nach einem beliebigen der Ansprüche 1 bis 11, wobei das Verhältnis von Piperacillin zu Tazobactam 8:1 beträgt.

13. Verfahren nach einem beliebigen der Ansprüche 1 bis 11, wobei das Verhältnis von Piperacillin zu Tazobactam 4:1 beträgt.

## Revendications

1. Procédé de production de Pipéracilline Sodique lyophilisée en combinaison avec du Tazobactame Sodique, avec une stabilité améliorée de la solution reconstituée, comprenant les étapes de :
i. dissolution de Pipéracilline acide en combinaison avec du Tazobactame dans l'eau au moyen d'une réaction avec du bicarbonate de sodium et/ou du carbonate de sodium,
ii. dégazage jusqu'à une teneur en dioxyde de carbone de moins de 200 mg/L,
iii. ajustement du pH à une valeur entre 5,6 et 7,9,
iv. lyophilisation.

2. Procédé selon la revendication 1 comprenant en outre une filtration stérile avant la lyophilisation.

3. Procédé selon l'une quelconque des revendications 1 à 2 dans lequel des quantités molaires approximativement équivalentes de base, à savoir de bicarbonate de sodium et/ou de carbonate de sodium, par rapport aux acides, à savoir la Pipéracilline acide en combinaison avec le Tazobactame acide, sont utilisées.

4. Procédé selon l'une quelconque des revendications 1 à 2 dans lequel des quantités molaires approximativement équivalentes de bicarbonate de sodium par rapport à la Pipéracilline acide et au Tazobactame acide, sont utilisées.

5. Procédé selon l'une quelconque des revendications 1 à 4 dans lequel les acides, à savoir la Pipéracilline acide en combinaison avec le Tazobactame, sont ajoutés à la base, à savoir le bicarbonate de sodium et/ou le carbonate de sodium, dissous dans l'eau à une température n'excédant pas 15°C.

6. Procédé selon l'une quelconque des revendications 1 à 4 dans lequel la Pipéracilline acide et le Tazobactame acide sont ajoutés au bicarbonate de sodium dissous dans de l'eau à une température entre 5 et 9°C.

7. Procédé selon l'une quelconque des revendications 1 à 6 dans lequel un dégazage jusqu'à une teneur en dioxyde de carbone de moins de 75 mg/L est appliqué.

8. Procédé selon l'une quelconque des revendications 1 à 6 dans lequel le dégazage est réalisé au moyen du vide à une pression absolue inférieure à 100 mbar.

9. Procédé selon l'une quelconque des revendications 1 à 6 dans lequel le dégazage est réalisé au moyen du vide à une pression absolue inférieure à 30 mbar.

10. Procédé selon l'une quelconque des revendications 1 à 6 dans lequel le pH est ajusté à une valeur entre 6,3 et 7,1.

11. Procédé selon l'une quelconque des revendications 1 à 10 dans lequel le rapport de la Pipéracilline sur le Tazobactame est dans la plage de 20:1 à 1:1.

12. Procédé selon l'une quelconque des revendications 1 à 11 dans lequel le rapport de la Pipéracilline sur le Tazobactame est 8:1.

13. Procédé selon l'une quelconque des revendications 1 à 11 dans lequel le rapport de la Pipéracilline sur le Tazobactame est 4:1.
